(19) Europäisches Patentamt
European Patent Office
Office européen des brevets

(11) Numéro de publication: **0 070 788 A1**

(12) **DEMANDE DE BREVET EUROPEEN**

(21) Numéro de dépôt: **82420095.0**

(22) Date de dépôt: **09.07.82**

(51) Int. Cl.³: **C 07 C 51/56**, C 07 C 53/12

(30) Priorité: **17.07.81 FR 8114124**

(43) Date de publication de la demande: **26.01.83 Bulletin 83/4**

(84) Etats contractants désignés: **AT BE CH DE FR GB IT LI LU NL SE**

(71) Demandeur: **RHONE-POULENC CHIMIE DE BASE, 25, quai Paul Doumer, F-92408 Courbevoie (FR)**

(72) Inventeur: **Gallucci, Jacques, 2, Boulevard Général de Gaulle, F-69600 Oullins (FR)**
Inventeur: **Gauthier-Lafaye, Jean, 21, rue Duguesclin, F-69006 Lyon (FR)**
Inventeur: **Perron, Robert, La Pécolière, F-69390 Charly (FR)**

(74) Mandataire: **Varniere-Grange, Monique et al, RHONE-POULENC RECHERCHES Centre de Recherches de Saint-Fons Service Brevets B.P. 62, F-69190 Saint-Fons (FR)**

(54) **Procédé de carbonylation de l'acétate de méthyle.**

(57) L'invention a trait à un procédé de carbonylation de l'acétate de méthyle en phase liquide, en présence: (a) d'une source de cobalt, (b) d'un cocatalyseur choisi parmi le chrome, le molybdène, le tungstène et leurs complexes de formule:

$[M(CO)_{6-x} L_x]$

dans laquelle 0 = x = 3 et L et $L_x$ sont des ligands respectivement donneurs de 1 ou x paire(s) d'électrons, (c) d'un iodure ionique de formule: $A^+ I^-$ dans laquelle $A^+$ est un cation choisi dans le groupe constitué par les cations onium quaternaire dérivant des éléments du groupe de l'azote et les cations des métaux alcalins, et (d) le cas échéant, d'un carboxylate de formule:
$A'^{n+} (OCOR)_n^-$ dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atoms de carbone, la quantité totale de composés halogénés présente dans le milieu réactionnel (exprimée en atome-gramme d'halogène et désignée par $X_T$) étant telle que le rapport atomique $X_T/(A^+ + n.A'^{n+})$ soit inférieur ou égal à 1.

Le procédé convient notamment à la préparation de l'anhydride acétique.

EP 0 070 788 A1

PROCEDE DE CARBONYLATION DE L'ACETATE DE METHYLE

La présente invention a pour objet un procédé de carbonylation de l'acétate de méthyle.

La présente invention a plus particulièrement pour objet un perfectionnement au procédé de carbonylation de l'acétate de méthyle, mettant en oeuvre un catalyseur à base de cobalt.

Le brevet américain n° 2 730 546 décrit la carbonylation de l'acétate de méthyle en anhydride acétique en présence d'un catalyseur choisi parmi les complexes du cobalt de formule générale :

$$[R_4A]_2\ CoX_4$$

dans laquelle X représente un atome de brome ou d'iode, A un atome d'azote ou de phosphore et R représente un radical alkyle inférieur, par exemple.

Ces complexes peuvent être formés in-situ en chargeant d'une part un halogénure du cobalt ($CoX'_2$) et d'autre part un halogénure d'ammonium (ou de phosphonium) quaternaire ($R_4AX$). La formation des complexes en question peut alors être représentée par la réaction suivante :

$$2(R_4AX) + CoX'_2 \longrightarrow [R_4A]_2\ CoX'_2X_2$$

Toutefois, l'efficacité de ces catalyseurs à base de cobalt paraît relativement faible. Ce type de procédé, dont l'intérêt de principe n'est pas contesté, n'a pu déboucher sur une réalisation industrielle.

Le brevet français n° 2 242 362 (correspondant aux demandes américaines n° 394 220 et n° 467 977, respectivement du 4 septembre 1973 et du 8 mai 1974) décrit un procédé de préparation de l'anhydride acétique en deux stades, dans lequel, lors d'une première étape, le bromure, ou de préférence l'iodure, de méthyle est carbonylé pour obtenir l'halogénure d'acétyle correspondant, cet halogénure d'acétyle étant à son tour mis à réagir sur de l'acétate de méthyle pour conduire, dans une deuxième étape, à l'anhydride acétique, ce qui correspond au schéma réactionnel suivant, dans le cas où l'iodure de méthyle est la matière de

départ :

- Etape 1 :

$$CH_3I + CO \longrightarrow CH_3COI$$

- Etape 2 :

$$CH_3COI + CH_3COOCH_3 \longrightarrow (CH_3CO)_2O + CH_3I$$

Comme ce schéma le fait apparaître clairement, l'iodure de méthyle, matière de départ de l'étape 1, est "régénéré" dans l'étape 2. L'étape 1 est avantageusement conduite en présence de catalyseur à base de rhodium ; l'étape 2 serait favorisée par la présence de lithium et/ou de chrome. Les deux étapes peuvent être réalisées dans une même zone réactionnelle qui renfermera alors de l'iodure de méthyle, de l'acétate de méthyle, du rhodium et le cas échéant du lithium et/ou du chrome, voire de l'iodure d'acétyle, zone dans laquelle on introduira également du monoxyde de carbone.

Le brevet américain n° 4 115 444 propose un perfectionnement de la technique décrite dans le brevet français précité, perfectionnement qui consiste à ajouter au milieu réactionnel un composé organique du phosphore ou de l'azote, dans lequel le phosphore ou l'azote est trivalent. Ce brevet confirme l'intérêt potentiel des systèmes catalytiques à base de rhodium, voire de palladium ou d'iridium, et de chrome dans cette réaction.

Le brevet français n° 2 303 788 (correspondant aux demandes américaines n° 556 750 et n° 654 661 respectivement du 10 mars 1975 et du 5 février 1976) montre que la présence d'une quantité importante d'hydrogène dans le milieu réactionnel défini ci-avant influe de manière notable sur l'orientation de la réaction. En effet on obtient dans ces conditions un mélange renfermant une proportion prépondérante d'acide acétique, des quantités variables de diacétate d'éthylidène, d'anhydride acétique et d'acétaldéhyde.

L'intérêt principal de ces procédés mettant en oeuvre des catalyseurs à base de rhodium, voire de palladium ou d'iridium, les systèmes basés sur le couple (rhodium-chrome) paraissant être les plus actifs, réside essentiellement dans la possibilité qu'ils offrent, d'accéder à l'anhydride acétique au départ d'acétate de méthyle en utilisant des pressions partielles de monoxyde de carbone plus basses que

celles requises dans les procédés antérieurs.

Néanmoins, les tentatives de développement de ce type de procédés, même sur une simple échelle pré-industrielle, se heurtent à des difficultés non-négligeables.

Une première série de difficultés réside dans le fait que les catalyseurs à base de rhodium ou de palladium, voir d'iridium, métaux extrêment rares et coûteux, se désactivent notamment lors des traitements nécessaires à la récupération du (ou des) produit(s) de la réaction. En raison du coût de ces catalyseurs il est indispensable de les régénérer. Par ailleurs les pertes en rhodium qui paraissent inévitables dans les divers points d'une unité industrielle grèvent lourdement l'économie d'un tel procédé.

Une seconde série de difficultés trouve sa source dans la présence, nécessaire au bon déroulement de la réaction et à la stabilisation du rhodium, de grandes quantités d'iodure de méthyle (ou d'acétyle) qui implique des risques importants de corrosion dans les divers points d'une installation industrielle. Par ailleurs l'iodure de méthyle et/ou certains de ses dérivés formés dans le milieu réactionnel sont responsables d'une contamination intolérable du (ou des) produit(s) réactionnel(s) qui nécessite la mise en oeuvre d'étapes supplémentaires pour éliminer les iodures dont la présence s'avère indésirable dans les produits de réaction. Ces dérivés iodés présents en grandes quantités non seulement dans les produits mais aussi dans divers effluents issus de la zone réactionnelle doivent, pour des raisons économiques évidentes, être récupérés ce qui implique des stades de traitement additionnels.

Les divers problèmes, délicats à résoudre, associés à ce type de procédés apparaîtront plus clairement à la lecture des brevets français n° 2 438 023 et n° 2 438 024 (correspondant respectivement aux demandes américaines n° 949 344 et n° 949 345 déposées le 6 octobre 1978) et du brevet américain n° 4 241 219.

Par ailleurs, il est bien connu que l'acétate de méthyle peut être obtenu par réaction de l'acide acétique et du méthanol, l'acide acétique pouvant être produit par carbonylation du méthanol et le méthanol pouvant être à son tour fabriqué par hydrogénation du monoxyde de carbone. Les réactions en cause sont susceptibles d'être représentées

comme suit :

$$CO + 2H_2 \longrightarrow CH_3OH \qquad (a)$$

$$CH_3OH + CO \longrightarrow CH_3COOH \qquad (b)$$

$$CH_3COOH + CH_3OH \longrightarrow CH_3COOCH_3 + H_2O \qquad (c)$$

La carbonylation de l'acétate de méthyle en milieu sensiblement anhydre permet d'obtenir l'anhydride acétique selon la réaction suivante :

$$CH_3COOCH_3 + CO \longrightarrow CH_3CO\text{-}O\text{-}OCH_3 \qquad (1)$$

Ainsi, l'intérêt d'un procédé de carbonylation de l'acétate de méthyle en anhydride acétique (1) apparaît clairement lorsqu'on considère également les réactions (a) à (c) ci-avant, puisque globalement cet ensemble revient à un procédé par lequel l'anhydride acétique est produit au départ de monoxyde de carbone et d'hydrogène.

D'autre part, le cobalt étant un métal courant, on conçoit aisément l'intérêt potentiel de son utilisation dans un procédé de carbonylation de l'acétate de méthyle. Il est donc étonnant de constater que toutes les solutions antérieures proposées récemment reposent sur la préconisation de systèmes catalytiques à base de rhodium.

Il a maintenant été trouvé de manière tout à fait surprenante qu'il est possible de carbonyler efficacement l'acétate de méthyle, en faisant appel à un système catalytique à base de cobalt.

La présente invention a donc pour objet un procédé de carbonylation de l'acétate de méthyle en phase liquide en présence :

- (a) d'une source de cobalt,
- (b) d'un cocatalyseur choisi parmi le chrome, le molybdène, le tungstène et leurs complexes de formule :

$$[M\,(CO)_{6-x}\,L_x]$$

dans laquelle M représente un atome de molybdène, de tungstène ou, de préférence de chrome, L représente une molécule (ligand) capable de donner une paire d'électrons à l'atome central M et x est un nombre entier positif ou nul et inférieur ou égal à 3, Lx pouvant par ailleurs représenter une molécule capable de donner x paire(s) d'électrons à l'atome central M,

- (c) d'un iodure ionique de formule :

$$A^{+} I^{-}$$

dans laquelle $A^{+}$ est un cation choisi dans le groupe constitué par les cations onium quaternaire dérivant des éléments du groupe de l'azote et les cations des métaux alcalins,

et - (d) le cas échéant, d'un carboxylate de formule :

$$A'^{n+} (OCOR)_n^{-}$$

dans laquelle n est égal à 1 ou 2 et $A'^{n+}$ a la signification donnée ci-avant pour $A^{+}$, $A'^{n+}$ et $A^{+}$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone, la quantité totale de composés halogénés présente dans le milieu réactionnel (exprimée en atome-gramme d'halogène et désignée par $X_T$ dans ce qui suit) étant telle que le rapport atomique $X_T/(A^{+} + n.A'^{n+})$ soit inférieur ou égal à 1.

Le présent procédé de carbonylation de l'acétate de méthyle est remarquable en divers points. En effet il a été trouvé de manière tout à fait inattendue que l'adjonction d'un cocatalyseur du type (b) précité au milieu réactionnel renfermant des ingrédients du type (a), du type (c) et, le cas échéant, du type (d) mentionnés ci-avant, permet d'obtenir notamment de l'anhydride acétique, avec une productivité horaire particulièrement élevée, alors que dans les mêmes conditions réactionnelles, le cobalt, seul, ne possède qu'une médiocre activité carbonylante dans la réaction envisagée et que le cocatalyseur seul, dans ces mêmes conditions, ne développe pratiquement aucune activité carbonylante.

La Demanderesse, sans pour autant être liée par une quelconque explication (ou mécanisme réactionnel), est conduite par ces faits surprenants à penser que l'activité carbonylante doit être attribuée, dans le présent procédé, à la source de cobalt qui, dans les conditions réactionnelles, se transformerait en une espèce catalytiquement active, dont la nature précise n'est pas totalement élucidée.

Le second constituant métallique du système (cocatalyseur du type (b) précité), qui ne possède en lui-même pratiquement aucune activité carbonylante, ne servirait apparamment qu'à augmenter l'activité

et/ou la concentration en espèce active du cobalt ou sa durée de vie le cas échéant. La raison de cette influence remarquable est inconnue mais pourrait cependant résider dans un changement du dégré d'oxydation et/ou de la disposition, voire de la nature, des coordinats de l'atome central de cobalt. En d'autres termes, le cocatalyseur aurait pour fonction de faciliter l'apparition dans le milieu réactionnel de la forme active du cobalt.

Par ailleurs il a été trouvé de manière tout à fait surprenante que, contrairement à l'enseignement de l'art antérieur, l'efficacité du présent procédé n'est pas liée à la présence de grandes quantités d'iodure de méthyle. Au contraire, lorsqu'on ajoute de l'iodure de méthyle lors de la mise en oeuvre du présent procédé, on observe une baisse notable de son efficacité.

La raison précise de l'influence négative de l'iodure de méthyle, phénomène en contradiction totale avec l'enseignement de l'art antérieur, est inconnue. La Demanderesse, sans pour autant être liée par une quelconque explication, suppose que l'iodure de méthyle, lorsqu'il est présent en quantités notables, rompt les équilibres complexes qui seraient notamment à l'origine de la très grande réactivité du cobalt dans les conditions décrites.

Par contre l'activité carbonylante du système précédemment défini n'est pas liée à la nature précise du ou des composés du cobalt chargés initialement. N'importe quelle source de cobalt susceptible de réagir avec l'oxyde de carbone dans le milieu de réaction pour donner des complexes carbonyles du cobalt peut être utilisée dans le cadre du présent procédé. Les sources typiques du cobalt sont par exemple le cobalt métallique finement divisé, des sels inorganiques de cobalt (nitrate, carbonate, halogénures ...) ou des sels organiques en particulier des carboxylates. Peuvent également être employés les cobalt-carbonyles ou hydrocarbonyles. Parmi les dérivés du cobalt convenant à la mise en oeuvre du procédé selon l'invention, on peut citer le formiate, l'acétate et plus particulièrement le dicobaltoctacarbonyle.

La quantité précise de cobalt engagée à la réaction n'est pas fondamentale. En général, on opère avec une quantité de cobalt telle que la concentration dans le milieu réactionnel exprimée en

milliatomes-grammes par litre (mAt-g/l) soit comprise entre 0,1 et 500 et de préférence entre 0,5 et 100 mAt-g/l.

Un avantage du présent procédé réside dans le fait qu'il est possible d'obtenir de bons résultats avec une faible concentration en cobalt.

Pour mettre en oeuvre le présent procédé il est également fait appel à un cocatalyseur choisi parmi le chrome, le molybdène, le tungstène et leurs complexes de formule :

$$[M\ (CO)_{6-x}\ L_x]$$

dans laquelle M représente un atome de molybdène, de tungstène ou, de préférence de chrome, L représente une molécule (ligand) capable de donner une paire d'électrons à l'atome central M et x est un nombre entier positif ou nul et inférieur ou égal à 3, Lx pouvant par ailleurs représenter une molécule capable de donner x paire(s) d'électrons à l'atome central M.

Si les métaux eux-mêmes conviennent à la mise en oeuvre du présent procédé, on préfère cependant faire appel à leurs complexes de formule ci-avant, qui s'avèrrent plus efficaces. A titre d'exemples de ligands L on peut citer les phosphines tertiaires, les amines tertiaires et les arsines tertiaires ; à titre d'exemple de ligands $L_2$ on peut citer le cyclooctadiène-1,5 et les diphosphines ; le cycloheptatriène, le benzène, le toluène, les xylènes, le mésitylène et l'acétylbenzène sont des exemples de ligands du type $L_3$.

On trouvera de plus amples informations sur les divers types de ligands $L_x$ et sur la préparation de ces complexes, dans la littérature spécialisée, par exemple, en se reportant à :

- Advances in Organometallic Chemistry volume 3 - pages 196 et suivantes - 1965 Academic Press,
- Organometaliic Chemistry Reviews - 1968 - pages 173 et suivantes,
- Journal of Organometallic Chemistry 124 (1977) pages 262-264.

Les composés hexacarbonyles du chrome, du molybdène et du tungstène étant à la fois disponibles et très efficaces seront

avantageusement utilisés dans le cadre du présent procédé. Selon une variante particulièrement avantageuse du présent procédé on fait appel au chrome hexacarbonyle.

La quantité de cocatalyseur à mettre en oeuvre dans le cadre du présent procédé, n'est pas fondamentale. De manière générale cette quantité sera telle que le rapport atomique M/Co, M ayant la signification précédente, soit compris entre 0,1 et 100, de préférence, ce rapport sera compris entre 0,5 et 50. De manière avantageuse ce rapport sera supérieur à 1.

Le procédé selon la présente invention nécessite également la présence d'un iodure ionique de formule :

$$A^+ I^-$$

dans laquelle $A^+$ est un cation choisi dans le groupe constitué par les cations onium quaternaire dérivant des éléments du groupe de l'azote et les cations des métaux alcalins.

Par cations onium quaternaire dérivés des éléments du groupe de l'azote on entend des cations formés à partir d'azote, de phosphore, d'arsenic ou d'antimoine et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical unique divalent.

Parmi ces composés la Demanderesse préconise l'emploi d'iodures de phosphonium (ou d'ammonium) quaternaire. Les cations de ces iodures peuvent être représentés de manière commode par les formules (I) à (III) ci-après :

$$R_1 - \overset{\displaystyle R_2}{\underset{\displaystyle R_4}{\overset{|}{\underset{|}{Q^+}}}} - R_3 \qquad (I)$$

dans laquelle Q représente un atome d'azote ou de phosphore, $R_1$, $R_2$, $R_3$ et $R_4$, pouvant être identiques ou différents représentent des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter

des radicaux alkyles linaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone ; deux des radicaux $R_1$ à $R_4$ pouvant éventuellement former ensemble un radical unique divalent - alkylène ou alcénylène - comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

$$R_5-\underset{\underset{R_6}{|}}{N^+}=C\begin{matrix}\diagup R_7\\ \diagdown R_8\end{matrix} \qquad \text{(II)}$$

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple ; $R_6$ et $R_7$ ou $R_8$ peuvent former ensemble un radical unique divalent - alkylène ou alcénylène - comportant 4 atomes de carbone et le cas échéant 1 ou 2 doubles liaisons éthylèniques, l'atome d'azote étant alors inclus dans un hétérocycle, pour constituer, par exemple, un cation pyridinium.

$$(R_9)_2\underset{\underset{R_5}{|}}{Q^+}-(CH_2)_y-\underset{\underset{R_5}{|}}{Q^+}(R_9)_2 \qquad \text{(III)}$$

dans laquelle $R_5$ et $Q^+$ ont la signification donnée précédemment, $R_9$ pouvant être identique à $R_5$ représente un radical alkyle ayant de 1 à 4 atomes de carbone ou un radical phényle, et y est un entier compris entre 1 et 10 inclus et de préférence entre 1 et 6 inclus. A titre d'exemple d'iodures d'ammonium quaternaire convenant à la mise en oeuvre du présent procédé on peut citer les iodures

de tétraméthylammonium
de triéthylméthylammonium
de tributylméthylammonium,
de triméthyl(n-propyl)ammonium,

de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylènammonium,
de N,N-diéthyl-triméthylènammonium,
de N,N-diméthyl-tétraméthylènammonium,
de N,N-diéthyl-tétraméthylènammonium,
de N-méthylpyridinium
de N-éthylpyridinium
de N-méthylpicolinium

A titre d'exemple d'iodures de phosphonium quaternaire convenant également à la mise en oeuvre du présent procédé, on peut citer les iodures

de tétraméthylphosphonium,
d' éthyltriméthylphosphonium,
de triméthylpentylphosphonium,
d' octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri(n-butyl)phosphonium,
de méthyl-tri(méthyl-2 propyl)phosphonium,
de méthyltricyclohexylphosphonium,
de méthyltriphénylphosphonium,

de méthyltribenzylphosphonium,
de méthyl-tri(méthyl-4 phényl)phosphonium,
de méthyltrixylylphosphonium,
de dièthylméthylphénylphosphonium,
de dibenzylméthylphénylphosphonium,
d' éthyltriphénylphosphonium,
de tétraéthylphosphonium,
d'éthyl-tri(n-propyl)phosphonium,
de triéthylpentylphosphonium,
d' éthyltriphénylphosphonium,
de n-butyl-tri(n-propyl)phosphonium,
de butyltriphénylphosphonium,
de benzyltriphénylphosphonium,
de (ß-phényléthyl)diméthylphénylphosphonium,
de tétraphénylphosphonium,
de triphényl(méthyl-4 phényl)phosphonium

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les iodures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou $R_6$) est également un radical alkyle ayant de 1 à 4 atomes de carbone, conviennent particulièrement bien.

En outre, parmi les iodures d'ammonium on préfère ceux dont les cations correspondent à la formule (I) dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les iodures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres

radicaux étant identiques et choisis parmi les radicaux phényle, tolyle ou xylyle.

Les iodures des métaux alcalins conviennent également à la mise en oeuvre de la présente invention. Toutefois dans le cas où de tels iodures sont mis en oeuvre il convient d'introduire également dans le milieu réactionnel un solvant choisi parmi la tétraméthylènesulfone, la tétraméthylurée, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone. Ce type de solvant est utilisé à raison de 10 à 50 % (en volume) du milieu réactionnel, bien que des proportions supérieures ou inférieures puissent être mises en oeuvre.

La quantité d'iodure ionique à mettre en oeuvre dans le cadre du présent procédé est en général telle que le rapport molaire $I^-/Co$ soit supérieur ou égal à 5 et de préférence, supérieur ou égal à 10. Il n'est pas utile que ce rapport dépasse la valeur de 200. On fixera avantageusement le rapport molaire $I^-/Co$ à une valeur comprise entre 15 et 100.

Comme indiqué en tête du présent mémoire, la présente invention peut être mise en oeuvre également en présence d'un carboxylate de formule :

$$A'^{n+}\ (OCOR)^-_n$$

dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone.

Ce mode d'exécution constitue une variante avantageuse du présent procédé lorsqu'on fait appel à un iodure alcalin. En effet les carboxylates en cause semblent limiter l'apparition in-situ d'iodure de méthyle (dont on a déjà signalé l'effet néfaste dans le présent procédé) susceptible de se former par réaction d'un iodure alcalin sur l'acétate de méthyle (matière de départ) selon le schéma réactionnel ci-après, la formation d'iodure de méthyle étant plus importante au départ d'iodure de lithium qu'au départ d'une quantité équivalente d'iodure de sodium ou de

potassium :

$$A^+I^- + CH_3COOCH_3 \longrightarrow CH_3I + A^+(OCOCH_3)^-$$

Il n'est pas nécessaire que le carboxylate $A'^{n+}(OCOR)^-_n$ dérive du même cation que l'iodure ionique utilisé. Le carboxylate est avantageusement un acétate.

On constatera que certains acétates peuvent être considérés comme le produit d'addition de l'acétate de méthyle et d'une phosphine, d'une amine, d'une arsine ou d'une stibine selon le schéma donné dans le cas particulier de la triphénylphosphine pour simplifier l'exposé :

$$CH_3COOCH_3 + (C_6H_5)_3 P \rightarrow (CH_3)(C_6H_5)_3 P^+ (OCOCH_3)^-$$

C'est la raison pour laquelle chaque mole de phosphine, d'amine, d'arsine ou de stibine, éventuellement chargée ou alimentée sera assimilée à un équivalent-gramme de cation $A'^{n+}$ dans la condition reliant la quantité totale d'halogène ($X_T$) à celle des cations $A^+$ et $A'^{n+}$ présente dans le milieu réactionnel.

Lorsqu'on utilise un carboxylate du type défini précédemment sa quantité est en général telle que le rapport molaire $A'^{n+}/A^+$ soit compris entre 0,01 et 20. De préférence, ce rapport sera compris entre 0,05 et 10, de manière avantageuse entre 0,1 et 5.

Selon une variante avantageuse, le présent procédé est mis en oeuvre également en présence d'hydrogène. Dans le cadre de cette variante la pression partielle d'hydrogène déterminée à 25 °C sera alors d'au moins 1 bar et de préférence d'au moins 3 bars, et encore mieux, d'au moins 10 bars.

En général, la pression partielle d'hydrogène mesurée à 25 °C ne dépassera pas 100 bars ; de manière avantageuse elle sera inférieure à 70 bars et, de préférence, inférieure à 50 bars.

Comme les praticiens le savent, la nature du produit obtenu est fonction de la teneur en eau du milieu réactionnel. Lorsqu'on opère dans un milieu réactionnel qui, compte-tenu des diverses contraintes industrielles, renferme le moins d'eau possible (milieu pratiquement anhydre) on obtient sélectivement l'anhydride acétique. Par contre lorsque le milieu renferme une proportion notable d'eau, on peut obtenir de l'acide acétique en une proportion correspondant à celle de l'anhydride acétique obtenu dans un milieu pratiquement anhydre. Dans la

mesure où il s'avère économiquement plus intéressant de produire de l'anhydride acétique, le présent procédé sera avantageusement conduit dans un milieu pratiquement anhydre.

Comme indiqué en tête du présent mémoire il est nécessaire que la quantité totale de composés halogénés présente dans le milieu réactionnel ($X_T$, exprimée en atome-gramme d'halogène) soit telle que le rapport (atomique) $X_T/(A^+ + n.\ A'^{n+})$ soit inférieur ou égal à 1.

Cette condition n'exclut pas la possibilité de faire appel à des halogènes ($X_2$), à des acides halogénés (HX), à des halogénures d'alkyle (RX), ou à des halogénures de cobalt ($CoX_2$), mais elle implique que si l'on charge ou alimente ces types de composés on devra nécessairement charger ou alimenter soit un carboxylate $[A'^{n+}\ (OCOR)_n^-]$ défini précédemment, soit une phosphine, une amine, une arsine ou une stibine, en quantité au moins équivalente à la quantité d'atomes-grammes d'halogène (X) introduite, le cas échéant, au moyen des composés halogénés mentionnés ci-avant. On admet que dans les conditions réactionnelles, les composés halogénés $X_2$, HX, et $CoX_2$ vont réagir sur l'acétate de méthyle pour donner naissance à un halogénure de méthyle, qui réagira à son tour (ainsi que les halogénures d'alkyle RX) sur la phosphine, l'amine, l'arsine ou la stibine pour former l'halogénure d'onium quaternaire correspondant.

C'est la raison pour laquelle, dans l'hypothèse où on fait appel aux composés halogénés de types $X_2$, HX, RX, et $CoX_2$, chaque mole de phosphine, d'amine, d'arsine ou de stibine introduite pour "neutraliser" en quelque sorte ces sources d'halogène sera considérée comme un équivalent-gramme de cation $A^+$ dans la condition reliant la quantité totale d'halogène ($X_T$) à celle des cations $A^+$ et $A'^{n+}$ présente dans le milieu réactionnel.

L'halogénure de méthyle $CH_3X$, au même titre que les halogénures d'alkyle (RX) peut par ailleurs réagir sur les carboxylates $[A'^{n+}\ (OCOR)_n^-]$ pour donner naissance aux halogénures ioniques correspondants $(A'^{n+}X_n^-)$.

Les phosphines, amines, arsines et stibines auxquelles on recourra, le cas échéant, peuvent être représentées par la formule (IV) ci-après :

R1
R2
R3
Q'
(IV)

dans laquelle Q' représente un atome de phosphore, d'azote, d'arsenic ou d'antimoine, et $R_1$ à $R_3$ ont la même signification que précédemment.

On recourt avantageusement à une phosphine, et plus particulièrement à la triphénylphosphine.

Selon une variante avantageuse le milieu réactionnel renferme outre de l'acétate de méthyle et les divers constituants du système catalytique, définis dans le présent mémoire un solvant choisi dans le groupe constitué par la tétraméthylènesulfone, la tétraméthylurée, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone et l'anhydride acétique.

La pression partielle du monoxyde de carbone mesurée à 25 °C est en général supérieure à 10 bars et, de préférence supérieure à 30 bars.

La température de réaction qui peut varier dans de larges limites est en général comprise entre 60 et 300 °C. On opère, de préférence, à une température comprise entre 80 et 240 °C, et de manière avantageuse à une température supérieure à 100 °C, gamme dans laquelle le système catalytique développe une efficacité optimale.

Un avantage du présent procédé réside dans la possibilité de carbonyler efficacement l'acétate de méthyle même avec une faible concentration de cobalt, de cocatalyseur et d'iodure ionique, sous une pression totale en température de l'ordre de 20 à 300 bars, qui est donc bien inférieure à celle habituellement préconisée pour des systèmes catalytiques à base de cobalt.

Un autre avantage du présent procédé réside dans la disparition des diverses contraintes associées à l'utilisation des systèmes catalytiques récemment proposés pour réaliser cette carbonylation.

Comme les spécialistes concernés le savent parfaitement bien, l'acétate de méthyle, matière de départ dans le présent procédé, peut être formé in-situ à partir de diméthyléther, aussi est-il tout à fait

possible dans le cadre de la présente invention, de charger ou d'alimenter du diméthyléther ou un mélange de diméthyléther et d'acétate de méthyle.

En fin d'opération, les produits obtenus peuvent être aisément séparés, par distillation fractionnée du mélange résultant, par exemple.

Les exemples ci-après illustrent l'invention sans toutefois en limiter le domaine ou l'esprit.

Dans les exemples ainsi que dans les essais témoins le mode opératoire utilisé est le suivant :

Dans un autoclave en tantale de 125 ml de capacité (sauf indications contraires), on charge de l'acétate de méthyle, un ou plusieurs solvants, le cas échéant, et les divers constituants du système catalytique. Après fermeture de l'autoclave on admet une pression de monoxyde de carbone et une pression d'hydrogène, respectivement désignées par P (CO) et P ($H_2$) (valeurs mesurées à 25 °C).

L'agitation, par un système de va-et-vient, est mise en route et l'autoclave est alors porté à la température choisie et désignée par T, en 25 minutes environ. La pression totale en température, désignée par P(T), est maintenue sensiblement à la valeur indiquée par des recharges successives de monoxyde de carbone renfermant au maximum 1 % (en volume) d'hydrogène. Après une durée de réaction désignée par t, on refroidit l'autoclave et on le dégaze. Le mélange réactionnel est alors analysé par chromatographie et potentiométrie.

Dans les exemples les conventions suivantes sont utilisées :

- AcOH : désigne l'acide acétique
- $Ac_2O$ : désigne l'anhydride acétique
- TMS : désigne la tétraméthylènesulfone
- NMP : désigne la N-méthylpyrrolidone
- Pr : désigne la productivité exprimée en gramme d'anhydride acétique par heure et par litre
- RR : désigne le nombre de moles d'anhydride acétique formées pour 100 moles d'acétate de méthyle chargées
- mAt-g : signifie milliatome-gramme
- mMol : signifie millimole

Toutes les pressions [P(CO), $P(H_2)$ et P(T)] sont exprimées en bars.

EXEMPLES 1 à 4 - ESSAI TEMOIN (a) :

Dans le tableau (I) ci-après on a rassemblé les conditions particulières et les résultats obtenus lors d'une série d'essais dont les conditions communes sont les suivantes :

On charge 40 ml d'acétate de méthyle, 5 ml d'acide acétique, du dicobaltoctacarbonyle, du chrome hexacarbonyle (sauf dans l'exemple 2 où l'on a utilisé de la poudre de chrome), et un iodure ionique dont la nature est indiquée dans le tableau I par les lettres A, B ou C désignant respectivement :

- l'iodure de méthyltriphénylphosphonium,
- l'iodure de méthyltributylammonium,

et - l'iodure de tétraéthylammonium.

T = 210 °C ; P(T) = 250 bars ; $X_T/(A^+ + n.A'^{n+}) = 1$ sauf mentions contraires.

TABLEAU I

| Ex n° | Co m At-g/l | Cr/Co | Iodure Ionique nature | Iodure Ionique $I^-/Co$ | P(CO) | $P(H_2)$ | t | $Ac_2O$ g | $Ac_2O$ RR |
|---|---|---|---|---|---|---|---|---|---|
| 1 | 20 | 6 | A | 15 | 130 | 10 | 30 | 7,45 | 14,6 |
| a | " | " | " | " | " | " | " | 1,48 | 2,9 |
| 2 | " | 70 | " | " | " | " | " | 2,13 | 4,2 |
| 3 | 44 | 4,5 | B | 9,1 | " | " | " | 3,35 | 6,5 |
| 4 | 46 | 4,3 | C | 8,7 | 139 | 1,4 | 20 | 4,67 | 9,1 |

Dans l'essai témoin (a) on a ajouté 4,4 mMol d'iodure de méthyle à la charge. $[X_T/(A^+ + n.A'^{n+}) = 1,29]$.

EXEMPLES 5 à 19 - Essais-témoins (b) à (e) :

Dans le tableau (II) ci-après, on a rassemblé les conditions particulières et les résultats obtenus lors d'une série d'essais dont les conditions communes sont les suivantes :

On charge 15 ml de solvant dont la nature est précisée dans le tableau (II), 30 ml d'acétate de méthyle ou un mélange de 25 ml d'acétate de méthyle et de 5 ml d'acide acétique, la présence de ce mélange étant indiqué par un signe + dans la colone AcOH du tableau II, du dicobaltoctacarbonyle, 15 mMol d'iodure de méthyltriphénylphosphonium (sauf dans les exemples 16 et 19 pour lesquels on a utilisé de l'iodure de sodium), et un cocatalyseur dont la nature et la quantité sont indiquées dans le tableau II.

T = 210 °C, t = 30 mn, P = 250 bars, $[X_T/(A^+ + n.A'^{n+}) = 1$ sauf mention contraire].

TABLEAU II

| Ex n° | AcOH | SOLVANT | Co mAt-g/l | COCATALYSEUR nature | COCATALYSEUR M/Co | P(CO) | $P(H_2)$ | $Ac_2O$ g | $Ac_2O$ Pr |
|---|---|---|---|---|---|---|---|---|---|
| b | + | NMP | 20 | - | 0 | 130 | 10 | 6,49 | 220 |
| c | " | " | " | $Cr(OAc)_3$ | 6 | " | " | 5,43 | 220 |
| 5 | " | " | " | $Cr(CO)_6$ | 0,5 | " | " | 7,60 | 305 |
| 6 | " | " | " | " | 3 | " | " | 10,32 | 410 |
| 7 | " | " | " | " | 6 | " | " | 12,55 | 500 |
| d | " | " | 0 | " | " | " | " | 0 | - |
| e | " | " | 20 | - | 0 | 139 | 1,4 | 3,15 | 125 |
| 8 | " | " | " | $W(CO)_6$ | 6 | " | " | 3,50 | 140 |
| 9 | " | " | " | $Mo(CO)_6$ | " | " | " | 4,03 | 160 |
| 10 | " | " | " | $Cr(CO)_6$ | " | " | " | 13,21 | 530 |
| 11 |  | - | " | " | " | " | " | 12,97 | 520 |
| 12 | + | TMS | " | " | " | " | " | 9,88 | 395 |
| 13 | - | " | " | " | " | " | " | 12,2 | 490 |
| 14 | + | " | " | " | " | 140 | 0 | 9,03 | 360 |
| 15 | - | " | " | " | " | " | " | 11,4 | 430 |
| 16 | + | " | 40 | " | 5 | 139 | 1,4 | 4,7 | 147 |
| 17 | + | " | 20 | " | 6 | " | " | 9,57 | 383 |
| 18 | + | " | 10 | " | 20 | " | " | 9,77 | 391 |
| 19 | + | " | 40 | " | 5 | " | " | 7,17 | 287 |

- l'Essai témoin d est réalisé en l'absence de cobalt et en présence de 15 mMol d'iodure de méthyltriphénylphosphonium.

- Dans l'exemple 19 on a ajouté à la charge, 10 mMol d'acétate de sodium. $[X_T/(A^+ + n.A'^{n+}) = 0,6]$.

- $I^-/Co$ est égal à 15 dans les exemples 5 à 15 et 17, ainsi que dans les essais témoins (c) à (e) ; dans les exemples 16 et 19 $I^-/Co$ = 7,5 et dans l'exemple 18 $I^-/Co$ = 30.

EXEMPLE 20 :

On reproduit l'exemple 1, décrit précédemment, en remplaçant 5 ml d'acétate de méthyle par un volume d'eau équivalent. En fin d'essai on dose 17,8 g d'acide acétique.

EXEMPLE 21 :

Dans un autoclave en Hastelloy B2 de 125 ml de capacité et selon le mode opératoire décrit précédemment, on réalise un essai sur une charge constituée de :

- 25 ml d'acétate de méthyle,
- 5 ml d'AcOH,
- 15 ml de NMP,
- 1 m At-g de cobalt sous forme de dicobaltoctacarbonyle (Co = 20 m At-g/l),
- 5 m At-g de chrome sous forme de chrome hexacarbonyle (Cr/Co = 5),
- 15 mMol d'iodure de méthyltriphénylphosphonium ($I^-/Co$ = 15),

Les conditions opératoires sont les suivantes :

- T = 210 °C,
- P(CO) = 77 bars,
- $P(H_2)$ = 1 bar
- P(T) = 150 bars.

En deux heures de réaction en température on a obtenu 12,9 g d'$Ac_2O$ (RR = 40,9 %).

EXEMPLE 22 - essai témoin (f) :

Dans un autoclave en Hastelloy B2 de 125 ml de capacité et selon le mode opératoire décrit précédemment on réalise deux essais sur une charge comprenant :

- 30 ml d'acétate de méthyle
- 20 ml de NMP
- 1,5 mAt-g de cobalt sous forme de dicobaltoctacarbonyle (Co = 30 mAt-g/l)

- 30 mMol id'iodure de lithium ($I^-/Co$ = 20).

Dans l'exemple 22, la charge renferme en outre 6 mMol de chromehexacarbonyle.

Les conditions communes sont les suivantes :

- T = 152 °C
- P(CO) = 32
- $P(H_2)$ = 7
- P(T) = 60

Pour chaque essai, on enregisitre l'absorption du monoxyde de carbone. Dans l'essai témoin (f) on constate qu'il n'y a plus d'absorption au bout de 40 minutes de réaction en témpérature ; l'essai est cependant poursuivi. Dans l'exemple 22 on ne constate pas d'arrêt de l'absorption pendant toute la durée de l'essai en température.

Les conditions particulières ainsi que les résultats obtenus sont rassemblés dans le tableau III ci-après.

TABLEAU III

| REFERENCE | EXEMPLE 22 | TEMOIN (f) |
|---|---|---|
| Cr (mAt-g) | 6 | 0 |
| t (mn) | 370 | 80 |
| $Ac_2O$ (g) | 12,5 | 6,3 |
| durée d'absorption (mn) | > 370 | 40 |

REVENDICATIONS

1. - Procédé de carbonylation de l'acétate de méthyle en phase liquide en présence :

- (a) d'une source de cobalt,

- (b) d'un cocatalyseur choisi parmi le chrome, le molybdène, le tungstène et leurs complexes de formule :

$$[M\ (CO)_{6-x}\ L_x]$$

dans laquelle M représente un atome de molybdène, de tungstène ou, de préférence, de chrome, L représente une molécule (ligand) capable de donner une paire d'électrons à l'atome central M et x est un nombre entier positif ou nul et inférieur ou égal à 3, Lx pouvant par ailleurs représenter une molécule capable de donner x paire(s) d'électrons à l'atome central M,

- (c) d'un iodure ionique de formule :

$$A^+\ I^-$$

dans laquelle $A^+$ est un cation choisi dans le groupe constitué par les cations onium quaternaire dérivant des éléments du groupe de l'azote et les cations des métaux alcalins,

et - (d) le cas échéant, d'un carboxylate de formule :

$$A'^{n+}\ (OCOR)^-_n$$

dans laquelle n est égal à 1 ou 2 et $A'^{n+}$ a la signification donnée ci-avant pour $A^+$, $A'^{n+}$ et $A^+$ pouvant par ailleurs être identiques ou différents, $A'^{n+}$ pouvant, en outre, représenter un cation alcalino-terreux, et R est un radical alkyle, aralkyle ou aryle ayant au maximum 8 atomes de carbone, la quantité totale de composés halogénés présente dans le milieu réactionnel (exprimée en atome-gramme d'halogène et désignée par $X_T$) étant telle que le rapport atomique $X_T/(A^+ + n.A'^{n+})$ soit inférieur ou égal à 1.

2. - Procédé selon la revendication 1, caractérisé en ce que M représente un atome de chrome.

3. - Procédé selon la revendication 1, caractérisé en ce que le cocatalyseur est choisi parmi les composés hexacarbonyles du chrome, du molybdène et du tungstène.

4. - Procédé selon l'une quelconque des revendications précédentes, caractérisé en ce que le cocatalyseur est le chrome hexacarbonyle.

5. - Procédé selon l'une quelconque des revendications précédentes, caractérisé en ce que l'on opère également en présence d'hydrogène.

6. - Procédé selon la revendication 1, caractérisé en ce que l'iodure ionique est un iodure de phosphonium quaternaire ou d'ammonium quaternaire.

7. - Procédé selon l'une quelconque des revendications précédentes, caractérisé en ce que la pression partielle du monoxyde de carbone mesurée à 25 °C est supérieure ou égale à 10 bars et, de préférence supérieure ou égale à 30 bars.

8. - Procédé selon l'une quelconque des revendications précédentes, caractérisé en ce que la température est comprise entre 60 et 300 °C et, de préférence entre 80 et 240 °C.

9. - Procédé selon l'une quelconque des revendications précédentes, caractérisé en ce que la concentration en cobalt est comprise entre 0,1 et 500 mAt-g/l.

10. - Procédé selon l'une quelconque des revendications précédentes, caractérisé en ce que le milieu réactionnel renferme un solvant choisi dans le groupe constitué par la tétraméthylènesulfone, la tétraméthylurée, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone et l'anhydride acétique.

11. - Procédé selon l'une quelconque des revendications 5 à 10, caractérisé en ce que la pression partielle d'hydrogène mesurée à 25 °C est inférieure à 100 bars et, de préférence, inférieure à 70 bars.

12. - Procédé selon l'une quelconque des revendications précédentes, caractérisé en ce que le rapport molaire $I^-/Co$ est supérieur ou égal à 5 et, de préférence supérieur ou égal à 10.

13. - Procédé selon l'une quelconque des revendications précédentes, caractérisé en ce que le rapport molaire $I^-/Co$ est compris entre 15 et 100.

14. - Procédé selon l'une quelconque des revendications précédentes, caractérisé en ce que le rapport atomique M/Co est compris entre 0,1 et 100.

15. - Procédé selon l'une quelconque des revendications précédentes, caractérisé en ce que le rapport atomique M/Co est compris entre 0,5 et 50.

16. - Procédé selon la revendication 14 ou 15, caractérisé en ce que le rapport atomique M/Co est supérieur à 1.

17. - Procédé selon l'une quelconque des revendications précédentes, caractérisé en ce que la concentration en cobalt est comprise entre 0,5 et 100 mAt-g/l.

18. - Procédé selon l'une quelconque des revendications 5 à 17, caractérisé en ce que la pression partielle d'hydrogène mesurée à 25 °C est inférieure à 50 bars.

0070788

Office européen des brevets

# RAPPORT DE RECHERCHE EUROPEENNE

Numéro de la demande

EP 82 42 0095

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int. Cl. 3) |
|---|---|---|---|
| Y | US-A-2 593 440 (HAGEMEYER) * revendication 1; colonne 3, lignes 13-19, 28-31, 36-41, 58-63, 68-75; colonne 4, lignes 3-12 * | 1,8,9, 14-17 | C 07 C 51/56<br>C 07 C 53/12 |
| Y | DE-A- 879 988 (B.A.S.F.) * revendications; page 2, lignes 2-11, 21-25 * | 1,8 | |
| A | DE-A- 857 192 (B.A.S.F.) * revendications; page 2, lignes 11-18; 28-33 * | 1,8 | |
| A | GB-A- 669 760 (BRITISH CELANESE) * revendication 1; page 2, lignes 13-23; page 3, lignes 114-122 * | 1 | DOMAINES TECHNIQUES RECHERCHES (Int. Cl. 3) |
| | | | C 07 C 51/00<br>C 07 C 53/00<br>C 07 C 67/00<br>C 07 C 69/00 |

Le présent rapport de recherche a été établi pour toutes les revendications

| Lieu de la recherche | Date d achevement de la recherche | Examinateur |
|---|---|---|
| LA HAYE | 07-10-1982 | KLAG M.J. |

CATEGORIE DES DOCUMENTS CITES

X : particulièrement pertinent à lui seul
Y : particulièrement pertinent en combinaison avec un autre document de la même categorie
A : arrière-plan technologique
O : divulgation non-écrite
P : document intercalaire
T : théorie ou principe à la base de l'invention
E : *document de brevet antérieur, mais publié à la date de depôt ou après cette date*
D : cite dans la demande
L : cité pour d'autres raisons
& : membre de la même famille, document correspondant

OEB Form 1503 03 82